# EUROPEAN PATENT SPECIFICATION

(11) **EP 2 982 982 B1**
(45) Date of publication and mention of the grant of the patent: **04.10.2017**
(21) Application number: 14290232.9
(22) Date of filing: 06.08.2014
(51) Int. Cl.: G01N 33/564, G01N 33/574, A61K 39/00

(54) **Method of screening of compounds using membrane STIM1**
Verfahren zum Screening von Verbindungen unter Verwendung von Membran-STIM1
Procédé de criblage de composés au moyen d'une membrane STIM1

(43) Date of publication of application: 10.02.2016
(73) Proprietor: Université de Bretagne Occidentale (U.B.O.), 29200 Brest (FR); Inserm, 75654 Paris Cedex 13 (FR); Centre Hospitalier Régional et Universitaire de Brest, 29609 Brest Cedex (FR)
(72) Inventor: Renaudineau, Yves, 29200 Brest (FR); Mignen, Olivier, 29460 Logonna Daoulas (FR); Burgos, Miguel, 48130 Massamagrell (Valencia) (ES); Pers, Jacques Olivier, 29200 Brest (FR); Fali, Tinhinane, 15000 Tizi-Ouzou (DZ)
(74) Representative: Novagraaf Technologies

(56) References cited:
- EP-A1- 2 103 311
- US-A1- 2014 187 616
- GEORGE C. TSOKOS: "Calcium signaling in systemic lupus erythematosus lymphocytes and its therapeutic exploitation", ARTHRITIS & RHEUMATISM, vol. 58, no. 5, 1 May 2008 (2008-05-01), pages 1216-1219, XP055163670, ISSN: 0004-3591, DOI: 10.1002/art.23445
- PERS J O ET AL: "Altered calcium signalling in B and T cells from Systemic Lupus Erythematosus patients is related to STIM1 expression", IMMUNOLOGY, WILEY-BLACKWELL PUBLISHING LTD, GB, vol. 137, no. Suppl.1, 1 September 2012 (2012-09-01), page 363, XP009181827, ISSN: 0019-2805
- MUKHERJEE SREYA ET AL: "Stromal interaction molecules as important therapeutic targets in diseases with dysregulated calcium flux", BIOCHIMICA ET BIOPHYSICA ACTA. MOLECULAR CELL RESEARCH, vol. 1843, no. 10, 25 March 2014 (2014-03-25), pages 2307-2314, XP029007393, ISSN: 0167-4889, DOI: 10.1016/J.BBAMCR.2014.03.019
- MASOOD AISHA ET AL: "Targeted treatment for chronic lymphocytic leukemia", ONCOTARGETS AND THERAPY, vol. 4, 2011, pages 169-183, XP9181847, ISSN: 1178-6930
- FATTAH ZOZIK ET AL: "Recent developments in the treatment of patients with systemic lupus erythematosus: focusing on biologic therapies", EXPERT OPINION ON BIOLOGICAL THERAPY, vol. 14, no. 3, March 2014 (2014-03), pages 311-326, XP9181848,
- FALI T ET AL: "The calcium sensor stromal interaction molecule 1 (STIM1) controls regulatory B cell functions and its activity is impaired in Systemic Lupus Erythematosus patients", ANNALS OF THE RHEUMATIC DISEASES, BRITISH MEDICAL ASSOCIATION, LONDON, GB, vol. 73, no. Suppl. 1, 1 January 2014 (2014-01-01), pages A49-A50, XP009185276, ISSN: 0003-4967
- Y. RENAUDINEAU ET AL: "A5.1 Abnormal calcium influx in T and B lymphocytes from systemic lupus erythematosus patients is related to STIM-1 overexpression.", ANNALS OF THE RHEUMATIC DISEASES, vol. 72, no. Suppl 1, 25 February 2013 (2013-02-25), pages A30-A31, XP55160196, ISSN: 0003-4967, DOI: 10.1136/annrheumdis-2013-203219.2

### SEQUENCE LISTING

<110> UBO
   INSERM
   CHU Brest
<120> Method of screening of compounds using membrane STIM1.
<130> BNT218446FR00
<160> 3
<170> PatentIn version 3.5
<210> 1
   <211> 685
   <212> PRT
   <213> Homo sapiens
<400> 1
<210> 2
   <211> 4062
   <212> DNA
   <213> Homo sapiens
<400> 2
   ctggacctgg gcaccgccag ccgcctgggc acgggactgg gcgggggcgc tgacctcggc 60
   ctaggaggcc caggatcccg gagacgcccg cgccctcagg accctgcggg tcgcacgccc 120
   tccccagctt ctgctgctcg ccgctcttcg gcagggcgag gtcaggtgcc cccttctcgc 180
   ctctcttctc ttctcttctc ttcctcctcc acttctgtgc ccgcggagac tccggccgcc 240
   cccttccgca ggggtgtagt aatctgcgga gctgacagca gccccgcagc caccctgccc 300
   gaagtctccg gaagcggcac gagctcaggc cgccgcagcc ccggcggacc cactgttgga 360
   cctgaggagc cagccctcct cccgcaccca aacttggagc acttgacctt tggctgttgg 420
   agggggcagg ctcgcgggtg gctggacagc tgcggagccg cgagggcatc ttgcctggag 480
   accgtcggct gcactcccgg gctcctggct ttgcctctgg gatcccgagg tgtccacatc 540
   agacgcatgt tgactgagac ctagagtcat ggatgtatgc gtccgtcttg ccctgtggct 600
   cctctgggga ctcctcctgc accagggcca gagcctcagc catagtcaca gtgagaaggc 660
   gacaggaacc agctcggggg ccaactctga ggagtccact gcagcagagt tttgccgaat 720
   tgacaagccc ctgtgtcaca gtgaggatga gaaactcagc ttcgaggcag tccgtaacat 780
   ccacaaactg atggacgatg atgccaatgg tgatgtggat gtggaagaaa gtgatgagtt 840
   cctgagggaa gacctcaatt accatgaccc aacagtgaaa cacagcacct tccatggtga 900
   ggataagctc atcagcgtgg aggacctgtg gaaggcatgg aagtcatcag aagtatacaa 960
   ttggaccgtg gatgaggtgg tacagtggct gatcacatat gtggagctgc ctcagtatga 1020
   ggagaccttc cggaagctgc agctcagtgg ccatgccatg ccaaggctgg ctgtcaccaa 1080
   caccaccatg acagggactg tgctgaagat gacagaccgg agtcatcggc agaagctgca 1140
   gctgaaggct ctggatacag tgctctttgg gcctcctctc ttgactcgcc ataatcacct 1200
   caaggacttc atgctggtgg tgtctatcgt tattggtgtg ggcggctgct ggtttgccta 1260
   tatccagaac cgttactcca aggagcacat gaagaagatg atgaaggact tggaggggtt 1320
   acaccgagct gagcagagtc tgcatgacct tcaggaaagg ctgcacaagg cccaggagga 1380
   gcaccgcaca gtggaggtgg agaaggtcca tctggaaaag aagctgcgcg atgagatcaa 1440
   ccttgctaag caggaagccc agcggctgaa ggagctgcgg gagggtactg agaatgagcg 1500
   gagccgccaa aaatatgctg aggaggagtt ggagcaggtt cgggaggcct tgaggaaagc 1560
   agagaaggag ctagaatctc acagctcatg gtatgctcca gaggcccttc agaagtggct 1620
   gcagctgaca catgaggtgg aggtgcaata ttacaacatc aagaagcaaa atgctgagaa 1680
   gcagctgctg gtggccaagg agggggctga gaagataaaa aagaagagaa acacactctt 1740
   tggcaccttc cacgtggccc acagctcttc cctggatgat gtagatcata aaattctaac 1800
   agctaagcaa gcactgagcg aggtgacagc agcattgcgg gagcgcctgc accgctggca 1860
   acagatcgag atcctctgtg gcttccagat tgtcaacaac cctggcatcc actcactggt 1920
   ggctgccctc aacatagacc ccagctggat gggcagtaca cgccccaacc ctgctcactt 1980
   catcatgact gacgacgtgg atgacatgga tgaggagatt gtgtctccct tgtccatgca 2040
   gtcccctagc ctgcagagca gtgttcggca gcgcctgacg gagccacagc atggcctggg 2100
   atctcagagg gatttgaccc attccgattc ggagtcctcc ctccacatga gtgaccgcca 2160
   gcgtgtggcc cccaaacctc ctcagatgag ccgtgctgca gacgaggctc tcaatgccat 2220
   gacttccaat ggcagccacc ggctgatcga gggggtccac ccagggtctc tggtggagaa 2280
   actgcctgac agccctgccc tggccaagaa ggcattactg gcgctgaacc atgggctgga 2340
   caaggcccac agcctgatgg agctgagccc ctcagcccca cctggtggct ctccacattt 2400
   ggattcttcc cgttctcaca gccccagctc cccagaccca gacacaccat ctccagttgg 2460
   ggacagccga gccctgcaag ccagccgaaa cacacgcatt ccccacctgg ctggcaagaa 2520
   ggctgtggct gaggaggata atggctctat tggcgaggaa acagactcca gcccaggccg 2580
   gaagaagttt cccctcaaaa tctttaagaa gcctcttaag aagtaggcag gatggggtgg 2640
   cagtaaaggg acagcttgtc cttccctggg tgttctgtct ctccttccct cccttccttc 2700
   aagataactg gccccaagag tggggcatgg gaagggctgg tccaggggtc tgggcactgt 2760
   acatacctgc cccctcatcc ttgggtcctt cattattatt tattaactga ccaccatggc 2820
   ctgcctgccc tgcctccgtc ccaaccatgg gctgctgctg tcactccctc tccacttcag 2880
   tgcatgtctt agttgctgtt ccctcagctc ccagctccac ctctggggtt cagcttctgt 2940
   ctctgctgtc ccagttttga ggtttggttt cttgtttctg tctcttgctt tcgggctcct 3000
   ccctcccacc actccccaac ttcccctagc agttgcaggg aagataggac gagtagcttc 3060
   tgacatgtgt gcctcagatc tgttccaccc cactcacagt ggttctgttt gctccagact 3120
   ggggctaggg cctaatcttt gaagtttgtt ctttggtatt gatgtgggtc agaaggagcc 3180
   tcatcctaat ctcactcagg cctccaggga tccatggggg agtgaaacca attctcagag 3240
   aacaacccac cagagacttt taaagagagg ccaggcttgg gaatgggttg ggagaggcat 3300
   ctgttcattg gagcatgagt ggatgccaga actgtaggtt ataaggcagt cactttttct 3360
   ctctactccc acccacacct gcctccctct tacccctgct cccccacact gcaggaggat 3420
   ttgtctctaa gaggtgctgc cccaaagctc cccaagcatc aatactccta gggctcagga 3480
   caagtggctc ccctggccag gagagccaca gccatgatac agggctctta tggagccctg 3540
   gagttgttgg gcaaggatgc tgtcattttt tgaaccaaaa gacaaacagg ttaaaaggaa 3600
   aaaaagtaat ctgaatttcc caagtgccta cgctgcatat tccccttgtt agatcccatt 3660
   ttcatgttac tttgtagcct tggccagagg ctcaaaaagg acacaaccag tttggggaag 3720
   gggtggctaa ggaagatggt ataggtgaag gcggctgtgt gaccactttc ccccaccctt 3780
   cccaccctct agacaactct ctcccttacc tgtttttgct atggctgtaa aggtattttt 3840
   cctctgcccc actccctgcc atacctttat cctgggatcc tattttgggc ctggggtggg 3900
   tatacctggg gctggtctta ggagggtgct aggctgcaga ctgccttgta ctccctggac 3960
   accctcaaat ggggttttct gtgttatttc ataaaattct ttgaagtcca ataaagcatg 4020
   taggagattt taaccactaa aaaaaaaaaa aaaaaaaaaa aa 4062
<210> 3
   <211> 191
   <212> PRT
   <213> Homo sapiens
<400> 3
   Leu Ser His Ser His Ser Glu Lys Ala Thr Gly Thr Ser Ser Gly Ala
   1               5                   10                  15
   Asn Ser Glu Glu Ser Thr Ala Ala Glu Phe Cys Arg Ile Asp Lys Pro
               20                  25                  30
   Leu Cys His Ser Glu Asp Glu Lys Leu Ser Phe Glu Ala Val Arg Asn
           35                  40                  45
   Ile His Lys Leu Met Asp Asp Asp Ala Asn Gly Asp Val Asp Val Glu
       50                  55                  60
   Glu Ser Asp Glu Phe Leu Arg Glu Asp Leu Asn Tyr His Asp Pro Thr
   65                  70                  75                  80
   Val Lys His Ser Thr Phe His Gly Glu Asp Lys Leu Ile Ser Val Glu
                   85                  90                  95
   Asp Leu Trp Lys Ala Trp Lys Ser Ser Glu Val Tyr Asn Trp Thr Val
               100                 105                 110
   Asp Glu Val Val Gln Trp Leu Ile Thr Tyr Val Glu Leu Pro Gln Tyr
           115                 120                 125
   Glu Glu Thr Phe Arg Lys Leu Gln Leu Ser Gly His Ala Met Pro Arg
       130                 135                 140
   Leu Ala Val Thr Asn Thr Thr Met Thr Gly Thr Val Leu Lys Met Thr
   145                 150                 155                 160
   Asp Arg Ser His Arg Gln Lys Leu Gln Leu Lys Ala Leu Asp Thr Val
                   165                 170                 175
   Leu Phe Gly Pro Pro Leu Leu Thr Arg His Asn His Leu Lys Asp
               180                 185                 190

## Description

### Field of technology

The present invention relates to the use of the membrane fraction of the STIM1 (stromal interaction molecule 1 or GOK) protein in a method of screening, as well as to a substance that interacts with the fraction of the STIM1 protein localized to the plasma membrane for therapeutic use and a pharmaceutical composition comprising at least this substance.

In the description given hereunder, the references in square brackets **([])** refer to the list of references given at the end of the text.

### Prior art

Systemic lupus erythematosus (SLE) and chronic lymphocytic leukaemia (CLL) are still incurable.

SLE is a heterogeneous disease, of autoimmune origin, characterized by the presence of autoreactive lymphocytes and of antinuclear auto-antibodies (ANA). It is a multisystemic disease, with very varied clinical manifestations. Prevalence varies in different ethnic groups, but is estimated at about 1 in 10000, with a male/female ratio of 10:1. The clinical heterogeneity of this disease reflects its aetiopathogenic complexity, comprising both genetic and environmental factors. SLE may affect all organs. The commonest manifestations are rash, arthritis and fatigue. The most severe manifestations include nephritis, neurological disorders, anaemia and thrombocytopenia. More than 90% of patients have ANAs that are considered positive above 1/160th. SLE is a disease with episodic evolution. The aims of the current treatment are: treat the acute episodes that may compromise the vital prognosis, minimize the risks of flare-ups during periods of relative stability and monitor the symptoms which, although not jeopardizing the vital prognosis, affect everyday quality of life.

Hydroxychloroquine and non-steroidal anti-inflammatories are indicated in the moderate forms of SLE; the corticoids and immunosuppressants are reserved for the most severe forms; the anti-CD20 monoclonal antibody (Rituximab, Mabthera®) that targets the B lymphocytes (B cells) is currently indicated in patients who are more severely affected and have not responded to the usual treatments **([1]).** Despite the improvement in prognosis after the introduction of corticoids and immunosuppressants, SLE continues to have a significant impact on patient morbidity and mortality.

CLL is a chronic malignant haemopathy that also affects the B cells. These cells play an important role at the immune system level. In the course of CLL, the B cells of CLL are blocked in their life cycle, when they reach maturity, and their production continues. Consequently, these B cells eventually accumulate in the blood, in the ganglia, spleen, liver and bone marrow, which leads to an increase in volume of the secondary lymphatic organs. The treatments currently available against CLL are most often used when the disease is at an advanced stage. The chemotherapeutic products used in the intensive treatment of CLL are chlorambucil used alone, fludarabine used alone, monthly chemotherapy of the CHOP type (combination of four agents: Cyclophosphamide-(H)adryamycin-Oncovin(vincristine)-Prednisone). In terms of targeted therapy, as the leukaemic B cells are CD20+, a monoclonal antibody specifically recognizing this target may be used in the treatment (rituximab, Mabthera®). Another target is Bruton's tyrosine kinase that is specific for the B cells whose expression is increased in the leukaemic cells. Ibrutinib, being an inhibitor of this enzyme, leads to apoptosis (death) of the leukaemic cells, giving longer remissions, even in the refractory or recurring forms. However, the treatments may give exposure to undesirable effects.

In the pathology of SLE and CLL, a disturbance of calcium signalling of the B cells in SLE and CLL is described following stimulation of the B-cell antigen receptor (BCR) **([2, 3]).**

In addition to these defects of calcium signalling, the B cells of SLE are characterized by a deficiency of production of interleukin 10 (IL-10), which affects the activity of the regulatory B lymphocytes (Bregs) **([4,5]).** This deficiency of activity of the Bregs in SLE leads to less regulation of T lymphocyte (T cell) proliferation, which might again contribute to amplifying the autoimmunity process **([5]).**

The diagnosis and prognosis of CLL and of SLE are based on a compilation of imperfect clinical and biological criteria, hence the need to develop new, more effective criteria.

In both of these disorders, the B cell represents the main therapeutic target. However, some patients do not respond to the existing treatments.

There is therefore a real need to offer new therapeutic solutions, which overcome these defects, drawbacks and obstacles of the prior art, and notably involve therapeutic targets that are readily accessible, specific and selective for the affected cells for the disease to be treated.

### Description of the invention

The invention makes it possible to respond to these needs by using the fraction localized to the plasma membrane, of the STIM1 protein, a protein involved in the activation and regulation of calcium channels, as therapeutic target of SLE and CLL.

The applicant has also demonstrated, surprisingly, that any means for direct or indirect control of the activity of the STIM1 protein localized to the plasma membrane may be used for modulating the cellular responses of the B cell, thus supplying a new therapeutic solution in SLE and CLL. The invention thus **describes** using, in this context, any tool that modulates (i) expression of the STIM1 protein localized to the plasma membrane, (ii) membrane addressing of this protein, or (iii) the biological activity of this protein at the lymphocyte membrane.

Y. Renaudineau (« Abnormal calcium influx in T and B lymphocytes from systemic lupus erythematosus patients is related to STIM-1 over-expression », ANNALS OF THE RHEUMATIC DISEASES, vol. 72, no. Suppl 1, 25 february 2013, pages A30-A31) and Fali et al. (« The calcium sensor stromal interaction molecule 1 (STIM1) controls regulatory B cell functions and its activity is impaired in Systemic Lupus Erythematosus patients », ANNALS OF THE RHEUMATIC DISEASES, vol. 73, No. Suppl 1, 1 January 2014, pages A49-A50) disclose that STIM1 is overexpressed in SLE, when compared with healthy controls. The overexpression of STIM1 is measured by Western blot and flow cytometry, on entire cells. So, in these documents, the whole STIM1 proteins cell content is detected, i.e. STIM1 proteins that are localized both at the plasma membrane and to the endoplasmic reticulum.

In the context of the present invention, the following are observed, surprisingly, in the B cells of patients with lupus:
(1) an increase in overall expression of the STIM1 protein and induction of the fraction of STIM1 localized to the plasma membrane, which remains low or even zero in the B cells of controls,
(2) activation of the MAPK pathway (mitogen-activated protein kinases) with phosphorylation of the Erk1/2 kinases (extracellular signal-regulated kinases) in the B cells of SLE possessing the fraction of STIM1 localized to the plasma membrane at rest, in particular in the B cells of SLE at the immature/transitional stage,
(3) an increased constitutive entry of extracellular Ca²⁺, and
(4) a correlation between the increase in expression of STIM1, the constitutive entry of extracellular Ca²⁺ and the disturbances of activation of the MAPK ERK1/2 pathway that may explain the general activation of the cell, survival of the auto-reactive B cells and therefore the autoimmunity process.

Regarding the activity of the Bregs of SLE, it is observed surprisingly, for the first time, in the context of the present invention:
(x1) that the deficient regulatory activity of the Bregs is linked to the increase in expression of the STIM1 molecule in the B cells of SLE,
(x2) that the blocking of the STIM1 molecule localized to the plasma membrane, effected specifically by a blocking antibody or non-specifically by an siRNA targeting STIM1 in the B cells of SLE, restores (i) the production of IL-10 by the Bregs of SLE, (ii) inhibition of proliferation of the T cells, and (iii) induction of the regulatory T cells. Blocking of STIM1 localized to the plasma membrane has no effect in healthy controls.

For the B cells of CLL, the applicant also observed, surprisingly:
(a) that the increase in baseline level of intracytoplasmic calcium was associated with an increase in survival of the B cells of CLL, activation of the MAPK Erk1/2 pathway, nuclear translocation of the transcription factors NFAT2 (Nuclear factor of activated T-cells) and STAT3 (Signal transducer and activator of transcription 3), as well as the synthesis of IL-10 **([5]),**
(b) that the increase in the constitutive entry of extracellular Ca²⁺ was regulated by the increase in STIM1 protein localized to the plasma membrane.
(c) That the presence of the STIM1 protein at the membrane (group I) or its absence (group II) allowed two groups of patients to be distinguished.
(d) That an anti-STIM1 antibody directed against the STIM1 molecule present at the plasma membrane was capable of greatly reducing the constitutive entry of extracellular Ca²⁺ into the B cells of CLL of group I and more weakly into the B cells of CLL of group II,
(e) That the combination of an anti-STIM1 antibody specifically targeting the fraction of the STIM1 protein localized to the plasma membrane with the anti-CD20 antibody (rituximab: RTX), was capable of restoring the apoptotic effect induced by RTX in the B cells of group I patients (presence of the STIM1 protein at the plasma membrane). Addition of the anti-STIM1 antibody has no effect on the apoptotic effect of anti-CD20 of the group II patients (absence of the STIM1 protein at the membrane).

The invention **describes** using the fraction of STIM1 localized to the plasma membrane as a new therapeutic target in SLE and CLL.

The invention also **describes** using modulators of the fraction of STIM1 localized to the plasma membrane in these disorders.

The invention **describes** to the use of the **isolated** fraction of STIM1 localized to the plasma membrane as a therapeutic target in SLE and CLL by modulating its presence or its activity. Thus, the invention **describes** , among other things, (i) inhibition of expression of the STIM1 molecule at the plasma membrane of the cells, (ii) inhibition of membrane addressing of this protein, and (iii) inhibition of the biological activity of the STIM1 protein present at the plasma membrane.

It is proposed, notably in CLL patients resistant to treatment with RTX, to block the activity of STIM1 localized to the plasma membrane by an anti-STIM1 antibody specifically targeting the fraction of the STIM1 protein localized to the plasma membrane. In fact, it is proposed that modulation of the inflows of Ca²⁺ depending on the fraction of STIM1 localized to the plasma membrane by the modulators of STIM1 such as an anti-STIM1 Ac would sensitize the cells to apoptosis induced by RTX.

The invention is advantageous on several points, notably the marker is only present on the affected cells, and not on the healthy cells, which allows a gain in specificity and in selectivity. Moreover, expression by the immune cells of the STIM1 protein at the level of the plasma membrane facilitates the accessibility of this target.

Thus, a first object of the invention relates to the use of the **isolated** fraction of the STIM1 protein localized to the plasma membrane of the cells in a**n *in vitro*** method for screening candidate molecules for treating SLE and/or CLL.

"Fraction of the STIM1 protein localized to the plasma membrane of the cells" means, in the sense of the present invention, the glycosylated fraction of the STIM1 protein localized to the plasma membrane of the cells. The STIM1 protein possesses two glycosylation sites, an asparagine in position 131 and another in position 171. Glycosylation of the STIM1 molecule is a necessary and obligatory process for addressing the STIM1 molecule at the surface of the cell **([7]).** This fraction has a molecular weight of about 90±2kDa, which makes it possible to distinguish it from the non-glycosylated form of STIM1 (84±2 kDa). The two forms are detectable by Western blotting. The human STIM1 molecule (Stromal Interacting Molecule; also called GOK) is a protein with sequence ID NO: 1 corresponding to the Uniprot sequence: Q13586 or NCBI: NP_003147.2. This protein is encoded by the sequence ID NO: 2, corresponding to the NCBI sequence: NM_003156.3 (mRNA transcript).

"Fraction of the STIM1 protein localized to the plasma membrane" means any biological product resulting from isolation of the STIM1 protein localized to the plasma membrane of the cells. Isolation may be performed by all the means known by a person skilled in the art, for example by using a detergent (for example a non-ionic or ionic surfactant such as Triton X-100 or Triton NI 01; or polyoxyethylene sorbitan esters), after differential centrifugation, or by an immuno-chemical or protein-chemical technique using a step of targeting the membrane proteins (antibody, Thermo scientific sulfo-NHS-SS-biotin), this list not being limiting.

"Cells" means, in the sense of the present invention, any cell expressing STIM1 at the level of the plasma membrane. Advantageously, the cells are immune cells. They may be, for example, B cells and T cells. Advantageously, the cells are B cells from patients with SLE or CLL.

"Method of screening" means, in the sense of the present invention, any method allowing identification of a substance interacting with the membrane fraction of the STIM1 protein or modulating its membrane expression. It may be any method known by a person skilled in the art, for example biological screening, for example a technique selected from the group comprising immunofluorescence, Western blot, immunoprecipitation, surface plasmon resonance (SPR), flow cytometry, video microscopy, study of calcium flows, enzyme-linked immunosorbent assay (ELISA), and confocal microscopy, or biophysical screening, for example by measuring the variations in intracellular calcium concentration by fluorescence.

"Candidate molecule" means, in the sense of the present invention, any molecule that interacts with the fraction of the STIM1 protein localized to the plasma membrane of the cells. The interaction may be of the type of fixation of the candidate molecule on the STIM1 protein localized to the plasma membrane of the cells. Alternatively, the interaction may be a modulation of the activity or expression of this protein. Modulation of the activity of the fraction of the STIM1 protein localized to the plasma membrane may be due to a modification of the insertion of STIM1 in the plasma membrane, or to a modification of its interaction with the proteins that are associated with it. Modulation of the activity of the protein may be reflected in a change of the calcium flows such as a change in the constitutive entry of intracellular calcium or a change in calcium influxes activated during stimulation of a receptor such as the calcium influxes dependent on the release of reserves (SOCE, store operated calcium influx). The modulation of expression may be an increase or a decrease in expression of the STIM1 protein localized to the plasma membrane relative to a level measured on the same cell or a comparable cell before application of the candidate molecule. The modulation of expression of the STIM1 protein may for example be linked to transcriptional modifications, epigenetic modifications or a modulation of the glycosylation process that is indispensable for membrane addressing of the STIM1 protein.

A second object of the invention relates to a substance that interacts with the fraction of the STIM1 protein localized to the plasma membrane of the cells, for use as a medicinal product in the treatment of SLE and/or CLL**, said substance being an antibody directed against a fragment of the STIM1 protein of sequence SEQ ID NO: 3.**

"Substance" means, in the sense of the present invention, any molecule displaying interaction of the type of fixation to the STIM1 protein localized to the plasma membrane or modulation of the activity or expression of this membrane fraction of the STIM1 protein, as defined above. The substance may be of natural or synthetic origin. It may be a protein produced chemically or by any method of bioengineering, such as purification. The substance may notably be identified by applying the method of screening as defined above.

The substance **is** an antibody directed against an extracellular fragment of the STIM1 protein localized to the plasma membrane of sequence SEQ ID NO: 3. This sequence corresponds to amino acids 23-213 of STIM1. It may be the anti-GOK/STIM1 antibody (Clone: 44, BD Biosciences reference 910954).

The invention further relates to a pharmaceutical composition comprising at least one substance as defined above any suitable pharmaceutically acceptable vehicle, comprising for example excipients and additives that facilitate formulation of the substance in preparations that may be used pharmaceutically, **and an anti-CD20 antibody**. The expression "pharmaceutically acceptable" encompasses any vehicle that does not interfere negatively with the efficacy of the substance for treating SLE or CLL, and that is not toxic to the host to whom or to which it is administered. In particular, suitable pharmaceutically acceptable vehicles for a composition according to the invention are vehicles that are suitable in particular for systemic application. Suitable pharmaceutically acceptable vehicles are well known in the prior art and are described for example in Remington Pharmaceutical Sciences (Mack Publishing Company, Easton, USA, 1985), a standard reference text in this field. It may be for example one or more components selected from sodium citrate, polysorbate 80, sodium chloride, sodium hydroxide, hydrochloric acid, and water for injection.

Advantageously, the composition according to the invention may find application as a medicinal product. Particularly advantageously, the composition of the invention may find application as a medicinal product in the treatment of SLE or of cancer such as CLL.

A pharmaceutical composition **comprising** any active principle that potentiates the effect of the substance as defined above **is described.** It may be **a** molecule associated with the protein complex regulating the calcium channels associated with the STIM1 protein such as the proteins Orai and TRPC.

**Anti-CD20** may be any anti-CD20 known in human or animal therapy, for example the IDEC-C2B8 antibody (Rituximab, distributed by Hoffman-La Roche in Europe. Drugbank DB00073 (BIOD00014, BTD00014), ofatumumab (Arzera, GlaxoSmithKline), tositumomab (GSK, DB00081, BIOD00085, BTD00085), obinutuzumab (Gazyva, Roche, DB08935, GA101), ibritumomab (Tiuxetan, IDEC Pharmaceuticals, DB00078, BIOD00069, BTD00069), ublituximab (LFB) or AME-133v (Lilly, LY2469298), this list not being limiting.

Other advantages may also become apparent to a person skilled in the art on reading the examples given below, illustrated by the appended figures, given for purposes of illustration.

### Brief description of the figures:

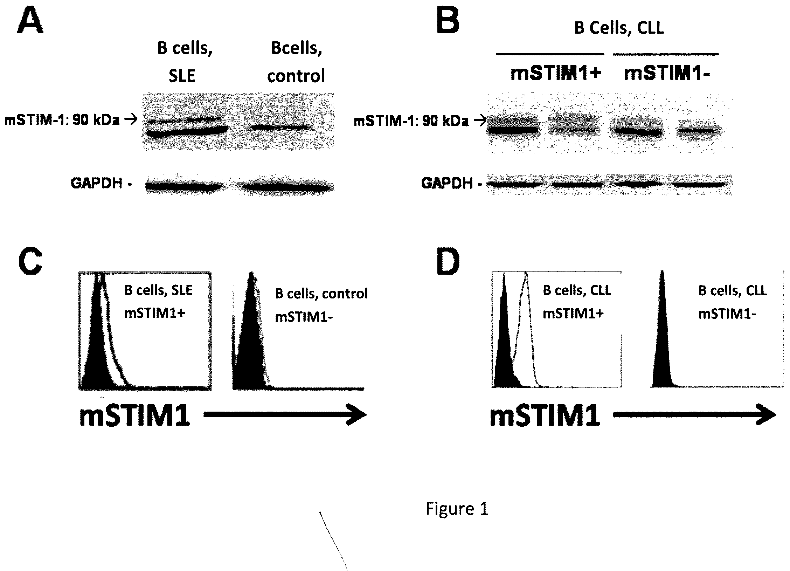

- **Figure 1** shows demonstration of membrane STIM1 in the B lymphocytes (B cells) of systemic lupus erythematosus (SLE) and the B cells of chronic lymphocytic leukaemia (CLL) by Western blot (A/B) and flow cytometry (C/D). Fig. A shows demonstration of a band at 90 kDa for the glycosylated fraction of STIM1 by Western blot in the B cells of SLE, versus the control B cells of healthy controls. This glycosylation of the STIM1 protein is indispensable for its insertion in the plasma membrane. Fig. B shows the demonstration by Western blot of a band at 90 kDa for the fraction of STIM1 localized to the membrane in the B cells of CLL expressing membrane STIM1 (mSTIM1+) versus the control CLL B cells not expressing membrane STIM1 (mSTIM1-). Fig. C shows demonstration by flow cytometry for the fraction of STIM1 localized to the membrane in the B cells of SLE expressing membrane STIM1 (mSTIM1+) versus the control B cells of healthy controls not expressing membrane STIM1 (mSTIM1-). Fig. D shows demonstration by flow cytometry for the fraction of STIM1 localized to the membrane in the B cells of CLL expressing membrane STIM1 (mSTIM1+) versus the control CLL B cells not expressing membrane STIM1 (mSTIM1-).

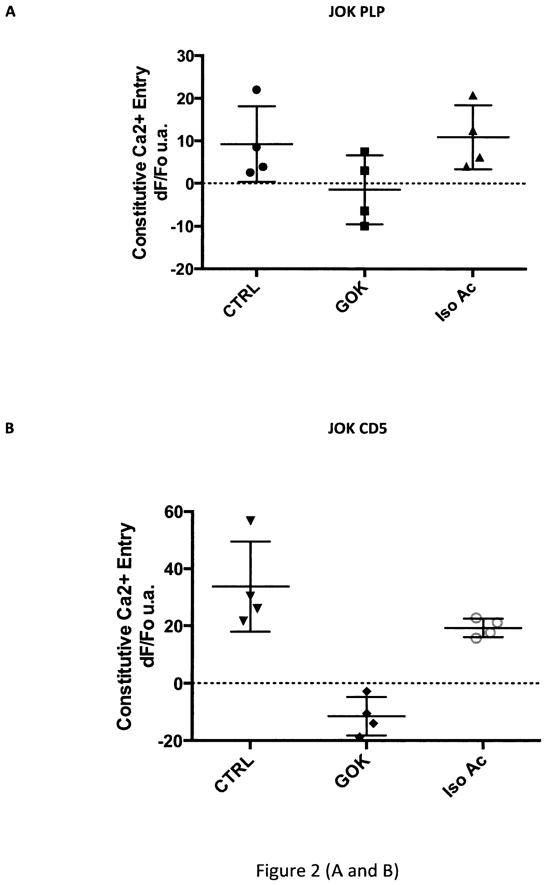

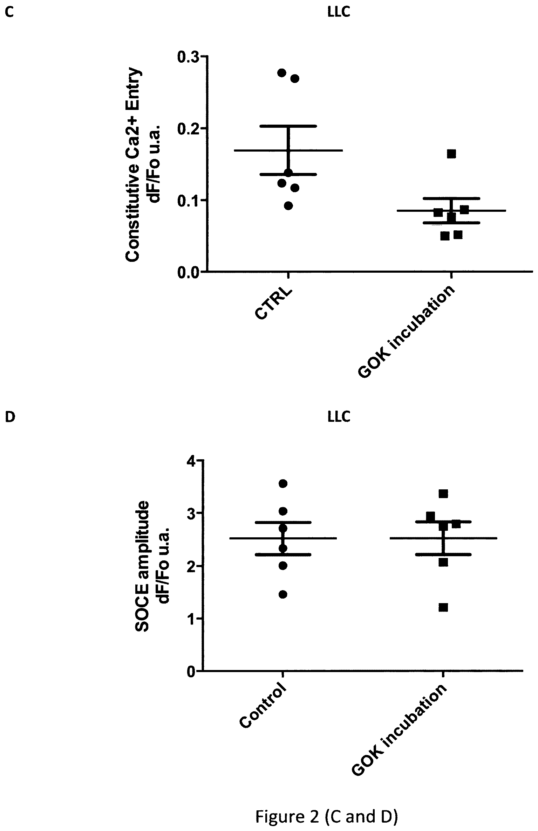

- **Figure 2** shows demonstration of the inhibition of the constitutive calcium influx by an anti-STIM1 antibody (clone Gok/44, BD Biosciences) directed against an extracellular epitope of the STIM1 protein localized to the plasma membrane B cells of the human line JOK PLP **([6]),** of the line JOK CD5 **([6]),** and B lymphocytes of chronic lymphocytic leukaemia (CLL). Figs. A and B show measurement of the constitutive influx and of the effects of the anti-STIM1 antibody on this constitutive influx (expressed in dF/Fo a.u., arbitrary units) measured in the B lymphocytes of the human lines JOK PLP (A) and JOK CD5 (B) in a multiwell plate using a plate reader for pretreated cells (5 µg/ml of antibody for 60 min) with the control antibody (CTRL, IgG2a isotype, Beckman Coulter) or with the anti-STIM1/GOK antibody. Fig. C shows measurement of the constitutive influx and of the effects of the anti-STIM1 antibody on this influx (as the ratio dF/Fo a.u.) on the B cells of CLL in single cell imaging for pretreated cells (5 µg/ml of antibody for 60 min) with the control antibody (CTRL, IgG2a isotype) or with the anti-STIM1/GOK antibody. Fig. D shows absence of an effect of the anti-STIM1 antibody on the calcium influx dependent on the release of reserves SOCE (store operated calcium influx) induced by thapsigargin (1 µM, Sigma-Aldrich) and measured in B cells of CLL.

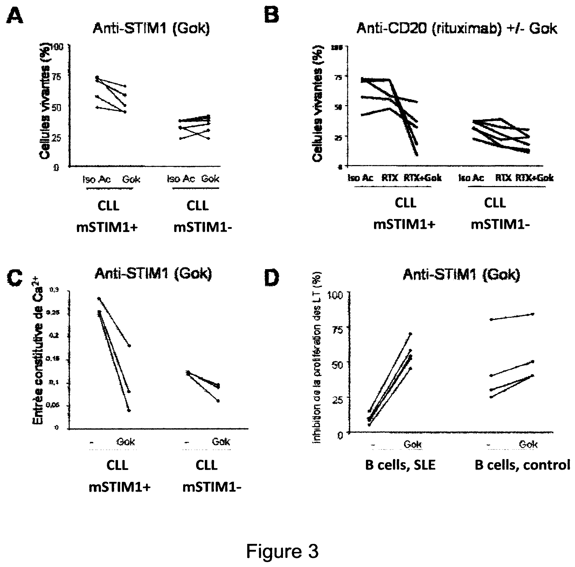

- **Figure 3** shows demonstration of the effects of the anti-STIM1 antibody (clone Gok/44) (A) on cellular viability alone or (B) in synergy with the anti-CD20 antibody (rituximab), (C) on constitutive calcium entry (Ca2+) in the B cells of chronic lymphocytic leukaemia (CLL) in patients classified in two groups depending on expression (mSTIM1+) or not (mSTIM1-) of the STIM1 protein at the plasma membrane and (D) on inhibition of the proliferation of T lymphocytes (Breg activity) by the B cells of systemic lupus erythematosus (SLE). Fig. A shows the percentage of live cells after 48h of culture for the B cells of CLL in the two groups mSTIM1+ or mSTIM1- in the presence of 10µg/ml of an isotypic control antibody (iso Ab, IgG2a isotype, Beckman Coulter) or in the presence of 10µg/ml of the anti-STIM1/GOK antibody. Fig. B shows the percentage of cells of CLL alive after 48h of culture for the B cells of CLL in the two groups mSTIM1+ or mSTIM1- in the presence of 10µg/ml of an isotypic control antibody (iso Ab, IgG2a isotype, Beckman Coulter), in the presence of 10µg/ml of rituximab (anti-CD20), or the combination rituximab (10 µg/ml) and anti-STIM1/GOK (10 µg/ml). Fig. C shows the reduction of constitutive entry of Ca²⁺ (expressed as the ratio dF/Fo a.u., arbitrary units) in the B cells of CLL of the group that expresses STIM1 at the plasma membrane (mSTIM1+) after pretreatment or not (control without addition) of the cells with 5 µg/ml of anti-STIM1/GOK antibody. Fig. D shows inhibition of proliferation of the cells expressed in percentage by the B cells of SLE in a model of autologous co-culture 1:1 after 4 days in the presence of an anti-STIM1/GOK antibody or of a control without addition.

### EXAMPLES

### Example 1: Method for detecting membrane STIM1

The B lymphocytes were purified starting from peripheral blood mononuclear cells (PBMC) obtained on a Ficoll gradient after removing the T lymphocytes (rosette technique using sheep red blood cells pretreated with neuraminidase) and monocytes (negative depletion technique, B cell kit without CD43, Stem Cell Technologies). The purity of the CD19-positive B cells was verified by flow cytometry, showing purity above 95%.

A/B- Protein analysis of the B cells by Western blot on SDS-PAGE made it possible to distinguish, in addition to the reticular fraction of STIM1 (84±2 kDa), the glycosylated membrane form of STIM1 (90±2 kDa) for the B cells of SLE (A) and for some CLL patients (B, mSTIM1+ group). This protein analysis used an anti-STIM1 clone Gok/44 antibody (BD Biosciences) first, then a peroxidase-linked mouse anti-IgG antibody (GE Healthcare), and finally detection by chemiluminescence (kit ECL advance, GE Healthcare).

C/D- Analysis by flow cytometry consisted of incubating the purified B cells with an anti-STIM1 clone Gok/44 antibody (BD Biosciences) for 15 min at 4°C, then, after washing, fixation of the anti-STIM1 antibody was revealed using a fluorescein-linked F(ab')2 mouse anti-IgG antibody (Jackson Laboratories). The membrane labelling of STIM1 in the live cells is determined relative to the isotypic control (IgG2a, Beckman Coulter).

### Example 2: Method of screening anti-membrane STIM1 molecules

Screening of the molecules modulating the STIM1 fraction localized to the plasma membrane is carried out to a first approximation on human B cell lines JOK that express the STIM1 protein at the plasma membrane. Two types of cells are used: JOK cells stably transfected with an empty vector (JOK PLP) or stably transfected with the CD5 protein **([6]).** These cells display a measurable constitutive calcium entry. Screening consists of measuring the effects of the molecules targeting the fraction of STIM1 localized to the plasma membrane of the cells on the constitutive entry of extracellular calcium. The effects of these molecules on calcium entry dependent on the release of reserves SOCE (Store Operated Calcium Entry) are also evaluated in order to determine the effect of the molecules on the influx SOCE of the molecules acting on constitutive calcium entry. The amplitude of these two calcium flows is measured by monitoring the variations in intracellular calcium concentration using a fluorescent probe (Calcium 6, Molecular Devices). The cells are made to adhere in 96-well plates treated with CellTak (BD Biosciences) at a rate of 100 000 cells per well for 45 minutes. The cells are then loaded with the fluorescent probe (Calcium 6, Molecular Devices) by incubation in the presence of this probe for 60 min before measuring the variations in intracellular calcium concentration using a multiwell plate reader of the Flexstation type (Molecular Devices). The cells are put in contact with the test compound at the moment of loading the cells with the fluorescent probe and throughout measurement of the variations in intracellular calcium concentration.

The measurement of constitutive calcium entry is estimated by removing and then adding the calcium of the extracellular matrix in the absence of any stimulation of the cells. The influx SOCE is activated by treating the cells with thapsigargin, a SERCA pump inhibitor.

The molecules identified as having an effect on the calcium flows of interest of the JOK cells are then tested on purified B cells obtained from peripheral blood mononuclear cells (PBMC) of control individuals or of CLL patients whose expression level of the STIM1 molecule at the surface of the cells is known and measured by flow cytometry. The effects of the test molecules on the constitutive calcium influx and the influx SOCE are measured as described above by single cell fluorescence imaging. The cells are made to adhere to glass slips coated with CellTAK (BD Biosciences) at a rate of 500 000 cells per slip for 45 min and loaded with the fluorescent probe (Fura2, Molecular probes) for 45 min in the presence of pluronic acid (Sigma Aldrich) before measuring the variations in intracellular calcium concentration using a single cell fluorescence imaging system. The cells are put in contact with the test compound throughout loading of the cells and throughout measurement of the variations in intracellular calcium concentration. The measurement of constitutive calcium entry is estimated by removing and then adding the calcium of the extracellular matrix in the absence of any stimulation of the cells. The influx SOCE is activated by treating the cells with thapsigargin, a SERCA pump inhibitor.

Inhibition of the constitutive calcium influx by an anti-STIM1 antibody (clone Gok/44, BD Biosciences) directed against an extracellular epitope of the STIM1 protein localized to the plasma membrane of the cells is thus demonstrated. The constitutive influx and the effects of the anti-STIM1 antibody on this influx are measured on the JOK lines in a multiwell plate and a plate reader (Figs. 2A/B) or on B lymphocytes in single cell imaging (Figs. 2C/D).

### Example 3: Methods for demonstrating biological and anti-lymphocyte B activity of the anti-membrane STIM1 molecules (figure 3)

**Fig 3A****-** The anti-STIM1 antibody (clone Gok/44, BD Biosciences) used at 10 µg/ml is capable of reducing the survival of the B cells of CLL, which was increased for the CLL of the mSTIM1+ group (presence of the STIM1 protein at the plasma membrane). For this experiment, the cells were cultured for 48h, then the percentage of live cells (absence of annexin V/propidium iodide labelling, Beckman Coulter) was determined.

**Fig 3B****-** The anti-STIM1 antibody (clone Gok/44) potentiates the action of the anti-CD20 antibody (rituximab, 10 µg/ml) on death of the B cells of CLL of the mSTIM1+ group.

**Fig 3C****-** The effect of the anti-STIM1 antibody (clone Gok/44) involves an effect of the antibody on the constitutive entry of Ca²⁺ in the B cells of CLL mSTIM1+.

**Fig 3D**- The anti-STIM1 antibody (clone Gok/44) restores the capacity of the B cells of SLE for inhibiting proliferation of the cells after 4 days of autologous culture in the presence of stimulation by CpG and anti-CD3/CD28.

### List of references

**[1]** Seret G, Hanrotel C, Bendaoud B, Le Meur Y and Renaudineau Y. Homozygous FCGR3A-158F mutation is associated with delayed B-cell depletion following rituximab but with preserved efficacy in a patient with refractory lupus nephritis. Clin Kidney J (2012) doi: 10.1093/ckj/sfs162.
**[2]** Nédellec S, Renaudineau Y, Bordron A, Berthou C, Porakishvili N, Lydyard PM, Pers JO, Youinou P. B cell response to surface IgM cross-linking identifies different prognostic groups of B-chronic lymphocytic leukemia patients. J Immunol. (2005)174:3749-56.
**[3]** Liossis SN, Kovacs B, Dennis G, Kammer GM, Tsokos GC. B cells from patients with systemic lupus erythematosus display abnormal antigen receptor-mediated early signal transduction events. J Clin Invest. (1996) 98:2549-57.
**[4]** Blair PA, Noreña LY, Flores-Borja F, Rawlings DJ, Isenberg DA, Ehrenstein MR, Mauri C. CD19(+)CD24(hi)CD38(hi) B cells exhibit regulatory capacity in healthy individuals but are functionally impaired in systemic Lupus Erythematosus patients. Immunity. (2010) 32:129-40.
**[5]** Lemoine S, Morva A, Youinou P, Jamin C. Human T cells induce their own regulation through activation of B cells. J Autoimmun. (2011) 36:228-38.
**[6]** Garaud S, Morva A, Lemoine S, Hillion S, Bordron A, Pers JO, Berthou C, Mageed RA, Renaudineau Y, Youinou P. CD5 promotes IL-10 production in chronic lymphocytic leukemia B cells through STAT3 and NFAT2 activation. J Immunol. (2011) 186:4835-44.
**[7]** Mignen O, Thompson JL, Shuttleworth TJ. STIM1 regulates Ca2+ entry via arachidonate-regulated Ca2+-selective (ARC) channels without store depletion or translocation to the plasma membrane. J Physiol. (2007) 579:703-15.

## Claims

1. Use of the isolated fraction of the STIM1 protein localized to the plasma membrane of the cells in an *in vitro* method for screening candidate molecules for treating chronic lymphatic leukaemia and/or systemic lupus erythematosus.

2. Use according to Claim 1, wherein the peptide sequence of the STIM1 protein is the sequence SEQ ID NO: 1.

3. Use according to Claim 1 or 2, wherein the method of screening uses a technique selected from the group comprising biological screening, and biophysical screening.

4. Use according to Claim 3, wherein screening uses a technique selected from the group comprising immunofluorescence, Western blot, immunoprecipitation, surface plasmon resonance (SPR), flow cytometry, video microscopy, study of calcium flows, enzyme-linked immunosorbent assay (ELISA), and confocal microscopy.

5. Substance interacting with the fraction of the STIM1 protein localized to the plasma membrane of the cells for use as a medicinal product in the treatment of chronic lymphatic leukaemia and/or systemic lupus erythematosus, said substance being an antibody directed against a fragment of the STIM1 protein of sequence SEQ ID NO: 3.

6. Pharmaceutical composition comprising at least one substance according to Claim 5, further comprising a pharmaceutically acceptable vehicle and an anti-CD20 antibody.

7. Pharmaceutical composition according to Claim 6, wherein the anti-CD20 antibody is the rituximab antibody.

## Patentansprüche

1. Verwendung der isolierten Fraktion des STIM1-Proteins, das an der Plasmamembran der Zellen lokalisiert ist, in einem *In-vi*tro-Verfahren zum Screening von Kandidatenmolekülen zum Behandeln von chronischer lymphatischer Leukämie und/oder systemischem Lupus erythematodes.

2. Verwendung nach Anspruch 1, wobei die Peptidsequenz des STIM1-Proteins die Sequenz SEQ ID NO: 1 ist.

3. Verwendung nach Anspruch 1 oder 2, wobei das Verfahren zum Screening eine Technik anwendet, die ausgewählt ist aus der Gruppe umfassend biologisches Screening und biophysikalisches Screening.

4. Verwendung nach Anspruch 3, wobei das Screening eine Technik anwendet, die ausgewählt ist aus der Gruppe umfassend Immunfluoreszenz, Western Blot, Immunpräzipitation, Oberflächenplasmonresonanz (SPR), Durchflusszytometrie, Videomikroskopie, Untersuchung von Calciumströmen, Enzymimmunoassay (ELISA) und konfokale Mikroskopie.

5. Stoff, der mit der Fraktion des STIM1-Proteins interagiert, das an der Plasmamembran der Zellen lokalisiert ist, zur Verwendung als Arzneimittel bei der Behandlung von chronischer lymphatischer Leukämie und/oder systemischem Lupus erythematodes, wobei der Stoff ein Antikörper ist, der gegen ein Fragment des STIM1-Proteins der Sequenz SEQ ID NO: 3 gerichtet ist.

6. Pharmazeutische Zusammensetzung, umfassend mindestens einen Stoff nach Anspruch 5, der ferner einen pharmazeutisch verträglichen Trägerstoff und einen Anti-CD20-Antikörper umfasst.

7. Pharmazeutische Zusammensetzung nach Anspruch 6, wobei der Anti-CD20-Antikörper der Rituximab-Antikörper ist.

## Revendications

1. Utilisation de la fraction isolée de la protéine STIM1 localisée à la membrane plasmique des cellules dans un procédé *in vitro* de criblage de molécules candidates pour le traitement de la leucémie lymphoïde chronique et/ou le lupus érythémateux systémique.

2. Utilisation selon la revendication 1, dans laquelle la séquence peptidique de la protéine STIM1 est la séquence SEQ ID NO : 1.

3. Utilisation selon la revendication 1 ou 2, dans laquelle le procédé de criblage utilise une technique choisie dans le groupe comprenant un criblage biologique, et un criblage biophysique.

4. Utilisation selon la revendication 3, dans laquelle le criblage utilise une technique choisie dans le groupe comprenant l'immunofluorescence, le Western blot, l'immunoprécipitation, la résonance des plasmons de surface (SPR), la cytométrie en flux, la vidéo microscopie, l'étude des flux calciques, la technique « enzyme linked immunosorbent assay » (ELISA), et la microscopie confocale.

5. Substance interagissant avec la fraction de la protéine STIM1 localisée à la membrane plasmique des cellules pour utilisation comme médicament dans le traitement de la leucémie lymphoïde chronique et/ou du lupus érythémateux systémique, ladite substance étant un anticorps dirigé contre un fragment de la protéine STIM1 de séquence SEQ ID NO : 3.

6. Composition pharmaceutique comprenant au moins une substance selon la revendication 5, et comprenant en outre un véhicule pharmaceutiquement acceptable et un anticorps anti-CD20.

7. Composition pharmaceutique selon la revendication 6, dans laquelle l'anticorps anti-CD20 est l'anticorps rituximab.
